# EUROPEAN PATENT APPLICATION

(11) **EP 4 835 066 A1**
(43) Date of publication of application: **04.11.2026**
(21) Application number: 24913416.4
(22) Date of filing: 26.12.2024
(51) Int. Cl.: C12N 9/24, C12N 15/81, C12P 19/04, C12P 19/14, C12R 1/84

(54) **MUTANT ENZYME FOR PRODUCING KESTOSE WITH HIGH YIELD**

(30) Priority: 29.12.2023 KR 20230197309
(71) Applicant: Samyang Corporation, Jongno-gu Seoul 03129 (KR)
(72) Inventor: KIM, Jeongmin, Yongin-si, Gyeonggi-do 16826 (KR); KIM, Minjeong, Yongin-si, Gyeonggi-do 16823 (KR); PARK, Busoo, Hanam-si, Gyeonggi-do 13014 (KR); AHN, Sinhye, Yongin-si, Gyeonggi-do 16827 (KR); LEE, Sanghee, Gwangju-si, Gyeonggi-do 12788 (KR); CHOI, Eunsoo, Seongnam-si, Gyeonggi-do 13588 (KR)
(74) Representative: Schön, Christoph
(86) International application number: PCT/KR2024/021170
(87) International publication number: WO 2025/143809

(57) **Abstract**

The present disclosure relates to: an enzyme for producing kestose, the enzyme having a high kestose conversion rate and a low by-product production rate; a strain expressing the enzyme; and a method of producing kestose by using the enzyme or the strain, whereby the purification cost of kestose can be reduced and high-purity kestose can be produced.

## Description

### Technical Field

The present disclosure relates to: a mutant enzyme for producing kestose, the mutant enzyme having a high kestose conversion rate and a low by-product production rate; a strain expressing the mutant enzyme; and a method of producing kestose by using the mutant enzyme or the strain, whereby the purification cost of kestose can be reduced and high-purity kestose can be produced.

### Background Art

Fructooligosaccharide (FOS) is an oligosaccharide in which one to three fructose are added to sugar, and is found in small amounts in nature in burdock, asparagus, and honeybees. Industrially, the FOS refers to a mixture of oligosaccharides consisting of kestose (sugar + fructose, GF2), nystose (sugar + fructose + fructose, GF3), and fructosylnystose (sugar + fructose + fructose + fructose, GF4), which are produced by a reaction using sugar and fructofuranosidase by Meiji, Japan.

The FOS is registered as health functional food with functions such as acting as a prebiotic that helps the growth of beneficial bacteria such as *Bifidobacterium*, regulating intestinal action, promoting calcium absorption, and the like. As concerns about high calories and adverse effects on blood sugar spikes are on the rise in modern society, interest is growing in the FOS, which is a sugar-derived substance that is low in calories and does not cause blood sugar spikes.

Industrial production of the FOS is currently carried out according to two strategies: partial degradation of inulin; or use of β-fructofuranosidase having high trans-fructosylase activity or use FTF mainly produced by fungi (*Aspergilllus niger*, *A. japonicus*, *A. oryzae*, *A. aculeatus*, and *Aureobasidium pullulans*) for the synthesis of an enzyme produced from sucrose. These two technologies produce FOS mixtures with varying degrees of polymerization (DP) from 2 to 10 and with the major components of 1-kestose (GF2), nystose (GF3), fructosylnystose (GF4), bifrucose (GF3), inulobiose (F2), inulotriose (F3), and inulotetraose (F4). From a commercial point of view, 1-kestose is the most valuable FOS due to its dual importance as a natural prebiotic sweetener and as a low-calorie sweetener useful as a sugar substitute for patients with diabetes.

However, the FOS is highly hygroscopic so that it cannot be a product manufactured in the crystalline form like sugar, and thus its application is limited as chocolate and the like. Fungal-derived beta-fructofuranosidase, an enzyme that produces the FOS from sugar, is characterized by high transfer activity and low isomer production, but its selectivity for oligosaccharide production is not as high as that of plant-derived sugar (sucrose): sucrose fructosyl transferase (SST), which produces only 1-kestose, and thus only a mixture of oligosaccharides with a DP of 2 to 6 may be prepared. Therefore, the FOS is available in liquid (syrup) or powder. However, such a form is an amorphous mixture such that there are issues such as high moisture absorption and poor processability as compared to crystalline food ingredients like sugar (sucrose).

It has been reported that kestose among the FOS components could be prepared in a form similar to sugar crystals, which may overcome the shortcomings of the FOS. Kestose exhibits physical properties of crystallinity similar to those of sugar, so thus it may be developed into a formulation that serves as a sugar substituted. It has been reported to have physiological properties such as reducing atopic dermatitis and inhibiting blood sugar spikes, in addition to the existing functional benefits of the FOS.

Because the existing FOS contains GF3, which cannot be crystallized, in the same ratio as GF2, the yield during the crystallization process was inevitably low. When mass-producing 1-kestose among the FOS, nystose or 1-F-fructofuranosylnystose, which is converted together with 1-kestose, has similar physical properties to those of 1-kestose, making its separation and purification significantly difficult. Therefore, to achieve industrially efficient large-scale production, it is necessary to develop a method of producing 1-kestose efficiently by improving enzymes, which are capable of selectively converting 1-kestose in excess relative to the other two components, or strains including the enzymes.

β-fructofuranosidase is an enzyme that recognizes fructose of sucrose and has the activity of hydrolyzing sucrose into fructose and glucose (sucrose-hydrolyzing activity). Among β-fructofuranosidases, those having the activity of converting fructose produced by hydrolysis into sucrose (fructose-transferring activity) and producing kestose, a trisaccharide composed of one molecule of glucose and two molecules of fructose also exist.

When producing kestose by using the β-fructofuranosidase, nystose, a tetrasaccharide, is typically produced as a by-product. Nystose is difficult to separate from kestose by chromatography, and thus is likely to remain in a reaction solution even after undergoing separation and purification by chromatography. In addition, a certain amount or more of nystose present in in the liquid inhibits crystallization of kestose during the crystallization process. For this reason, reducing the production of nystose is necessary for efficient preparation of kestose. Therefore, there is a need for the β-fructofuranosidase that can efficiently produce kestose with a low rate of producing by-products such as nystose.

### Disclosure of Invention

### Technical Problem

The purpose of the present disclosure is to provide an enzyme protein for producing kestose having a high kestose conversion rate and a low by-product production rate, a nucleic acid molecule encoding the enzyme protein, a recombinant vector including the nucleic acid molecule, or a transformant including the nucleic acid molecule or the recombinant vector.

Another object of the present disclosure is to provide a composition for producing a sugar conversion product including kestose from a substrate including sucrose, the composition including at least one selected from the group consisting of the enzyme protein, the recombinant microorganism, a cell of the microorganism, a cell lysate of the microorganism, a culture of the microorganism, a supernatant obtained from the culture, and an extract thereof.

Another object of the present disclosure is to provide a method of producing a sugar conversion product including kestose with a high conversion rate and a high content, the method including reacting a substrate including sucrose with at least one selected from the group consisting of the enzyme protein, a recombinant microorganism expressing the enzyme protein, a cell of the microorganism, a cell lysate of the microorganism, a culture of the microorganism, a supernatant obtained from the culture, and an extract thereof.

Another object of the present disclosure is to provide a sugar conversion composition (e.g., FOS) including a high content of kestose (e.g., 1-kestose) produced biologically from a substrate including sucrose.

Another object of the present disclosure is to provide a recombinant microorganism, such as yeast and mold, expressing an enzyme having a high kestose conversion rate and a low by-product production rate.

Another object of the present disclosure is to provide use of the composition for producing a sugar conversion product including kestose from a substrate including sucrose, the composition including at least one selected from the group consisting of the enzyme protein, a recombinant microorganism expressing the enzyme protein, a cell of the microorganism, a cell lysate of the microorganism, a culture of the microorganism, a supernatant obtained from the culture, and an extract thereof.

Another object of the present disclosure is to provide use of the enzyme protein, a recombinant microorganism expressing the enzyme protein, a cell of the microorganism, a cell lysate of the microorganism, a culture of the microorganism, a supernatant obtained from the culture, and an extract thereof, for preparing a composition for producing a sugar conversion product including kestose from a substrate including sucrose.

### Solution to Problem

The present disclosure relates to an enzyme for producing kestose having a high kestose conversion rate and a low by-product production rate, an enzyme protein including an amino acid sequence of the enzyme, a nucleic acid molecule encoding the enzyme protein, a recombinant vector including the nucleic acid molecule, a recombinant microorganism expressing the enzyme, and a method of producing kestose by using the enzyme protein and/or the recombinant microorganism.

The method of producing kestose according to the present disclosure involves a method of producing high-purity kestose, whereby the purification cost of kestose may be reduced. The enzyme is a mutant enzyme of beta-fructofuranosidase (FFase), and the FFase typically includes a polypeptide encoded by the *fopA* gene. An example of the polypeptide encoded by the *fopA* gene may be a mature protein including a signal peptide of SEQ ID NO: 2 or not including a signal peptide of SEQ ID NO: 1.

Specifically, the inventors of the present disclosure studied for improving the FFase to maximize a ratio of kestose (GF2) and nystose (GF3) in an FOS conversion reaction by the FFase, and accordingly developed a technology for mass production of kestose. In addition, an enzyme that produces kestose in a high content was produced by introducing a gene encoding a mutant enzyme into a strain of the genus *Pichia*, which mainly belongs to the yeast family.

Hereinafter, the present disclosure will be described in more detail.

### Mutant enzyme protein having β-FFase activity

The enzyme protein according to the present disclosure having a high kestose conversion rate and a low by-product production rate may be produced by introducing a mutation in at least one amino acid at a specific position in an amino acid sequence of the enzyme protein of a β-fructofuranosidase enzyme protein, for example, an amino acid sequence having at least 90 % sequence identity or homology with the amino acid sequence, wherein the mutation may preferably be an amino acid substitution.

Specifically, the amino acid mutation is deletion, substitution, insertion, or addition of one or multiple amino acids, and the number of amino acids to be deleted, substituted, inserted, or added may be, for example, 1 to 200, 1 to 180, 1 to 160, 1 to 140, 1 to 120, 1 to 100, 1 to 80, preferably 1 to 60, more preferably 1 to 50, even more preferably 1 to 40, and even most preferably 1 to 30.

The FFase according to an embodiment of the present disclosure may be derived from a peptide including the amino acid sequence of SEQ ID NO: 3 derived from a strain of the genus *Aspergillus* (specifically, *A. niger*) (Accession No: KCTC13139 BP). The amino acid sequence of SEQ ID NO: 3 is a mature enzyme protein, excluding the 19-amino acid signal sequence (MKLTTTTLALATGAAAAEA) from the FFase enzyme protein, and an immature protein derived from the strain and including a signal peptide with the amino acid sequence of the enzyme protein shown in SEQ ID NO: 4*. A. niger* derived from the circular enzyme protein may be a strain deposited with the KCTC on October 28, 2016, and assigned the Accession No: KCTC 13139BP.

The enzyme protein according to the present disclosure may be one in which at least one amino acid is mutated, for example substituted, in an amino acid sequence having at least 90 % sequence identity with the amino acid sequence of SEQ ID NO: 1, and may have FFase activity. Preferably, the enzyme protein has a high kestose conversion rate and a low by-product production rate.

The present disclosure provides the enzyme protein in which an amino acid sequence corresponding to at least one amino acid selected from the 62nd, 220th, 313th, 329th, and 440th amino acids from the N-terminus of the amino acid sequence of SEQ ID NO: 1 is mutated, and which has FFase activity.

Specifically, in an embodiment, the enzyme protein may have an amino acid sequence having at least 90 %, 91 %, 92 %, 93 %, 94 %, 95 %, 96 %, 97 %, 98 %, 99 %, 99.3 %, or 99.5 % sequence identity with the amino acid sequence of SEQ ID NO: 1, in which an amino acid corresponding to at least one amino acid selected from the group consisting of the 62nd, 220th, 313th, 329th, and 440th amino acids from the N-terminus of the amino acid sequence of SEQ ID NO: 1 is mutated, and may have FFase activity and preferably have a high kestose conversion rate and a low by-product production rate. Such comparison of identity and homology may be performed by calculating the identity and homology between two or more sequences as a percentage (%) by using a commercially available computer program. The enzyme protein may exclude an enzyme protein having the amino acid sequence of SEQ ID NO: 1.

It is obvious that, if a protein has an amino acid sequence having the identity or homology mentioned in the present disclosure and exhibits activity corresponding to the enzyme protein, proteins having amino acid sequences to which some sequences are deleted, modified, substituted, or added may also be included within the scope of the present application. That is, even if the present application describes "a protein having or including the amino acid sequence of a specific SEQ ID NO.", it is obvious that proteins having amino acid sequences to which some sequences are deleted, modified, substituted, or added may also be used in the present application as long as the proteins have the same or corresponding activity as a protein consisting of the amino acid sequence of the corresponding SEQ ID NO.

In the present disclosure, the "corresponding amino acid" refers to an amino acid residue at a corresponding position in an enzyme protein having FFase activity or an amino acid residue that is similar, identical, or homologous to the amino acid residue at the corresponding position. Identifying the amino acid at the corresponding position may include determining a specific amino acid of a sequence that references a specific sequence. In the present disclosure, the "corresponding position" generally refers to a similar or corresponding position in the amino acid sequence of the related protein or in a reference sequence. For example, any amino acid sequence may be aligned with SEQ ID NO: 3, and on this basis, each amino acid residue of the amino acid sequence may be numbered by referring to the numerical position of the amino acid residue corresponding to the amino acid residue of SEQ ID NO: 3. For example, through sequence alignment algorithms known in the art, positions of corresponding amino acids, or positions where modifications such as substitutions, insertions, or deletions occur may be identified by comparing with a query sequence (also referred to as a "reference sequence").

The amino acid mutation position is based on the amino acid sequence of SEQ ID NO: 1, which is the mature form, and may be the 81st, 239th, 332nd, 348th, or 459th amino acid based on the amino acid sequence of SEQ ID NO: 2, which is the immature form of the enzyme protein including the signal peptide.

The mutant amino acid sequence may involve amino acid sequence substitutions at the amino acid positions corresponding to at least one amino acid selected from the group consisting of the 62nd, 220th, 313th, 329th, and 440th amino acids from the N-terminus of the amino acid sequence of SEQ ID NO: 1, wherein the substituted amino acid may be, for example, substituted with at least one amino acid selected from the group consisting of tryptophan (Trp; W), alanine (Ala; A), lysine (Lys; K), and threonine (Thr; T).

Specifically, in an embodiment, the substituted amino acid sequence may include at least one (e.g., 1, 2, 3, 4, or 5) selected from the group consisting of a substitution of an amino acid corresponding to the 62nd glycine (Gly; G) with Trp, a substitution of an amino acid corresponding to the 220th valine (Val; V) with Ala, a substitution of an amino acid corresponding to the 313th histidine (His; H) with Lys, a substitution of an amino acid corresponding to the 329th glutamic acid (Glu; E) with Lys, and a substitution of an amino acid corresponding to the 440th arginine (Arg; R) with Thr, in the amino acid sequence of SEQ ID NO: 1 or an amino acid sequence having at least 90 %, 91 %, 92 %, 93 %, 94 %, 95 %, 96 %, 97 %, 98 %, 99 %, 99.3 %, or 99.5 % homology or identity with the amino acid sequence of SEQ ID NO: 1. However, the embodiment is not limited thereto.

In an embodiment of the present disclosure, the enzyme protein may include at least one amino acid substitution selected from the group consisting of G62W, V220A, H313K, E329K, and R440T, in the amino acid sequence of SEQ ID NO: 1.

In an embodiment of the present disclosure, the enzyme protein may include one amino acid substitution or at least two amino acid substitutions selected from the group consisting of G62W, V220A, H313K, E329K and R440T. For example, the enzyme protein in which one amino acid is substituted may include G62W, V220A, H313K, E329K, or R440T in the amino acid sequence of SEQ ID NO: 1, and specifically may include and/or may be represented by the amino acid sequence of SEQ ID NO: 6 or SEQ ID NO: 7.

The enzyme protein in which two amino acids are substituted may include two amino acid substitutions selected from the group consisting of G62W, V220A, H313K, E329K, and R440T (e.g., G62W/V220A, G62W/H313K, G62W/E329K, G62W/R440T, V220A/H313K, V220A/E329K, V220A/R440T, H313K/E329K, H313K/R440T, or E329K/R440T). Examples of the enzyme protein having three amino acid substitutions include G62W/V220A/H313K, G62W/V220A/H313K, G62W/V220A/E329K, G62W/V220A/E329K, G62W/V220A/R440T, G62W/V220A/R440T, G62W/H313K/E329K, G62W/H313K/R440T, V220A/ H313K/E329K, V220A/H313K/E329K, V220A/H313K/R440T, and the like, and an example of a mutant enzyme including three mutant amino acids is a peptide corresponding to G62W/H313K/R440T, which may be represented by the amino acid sequence shown in SEQ ID NO: 7. Examples of enzyme proteins containing four amino acid substitutions include G62W/V220A/H313K/E329K, G62W/V220A/H313K/R440T, G62W/H313K/E329K/R440T, V220A/H313K/E329K/R440T, and an example of a mutant enzyme containing four mutant amino acids may be represented by the amino acid sequence shown in SEQ ID NO: 6.

In an embodiment of the present disclosure, by using a method of increasing a low crystal yield, the content ratio of kestose/nystose (e.g., 1-kestose/nystose) based on the wt% of sugar solid content included in a reaction product, for example, the content ratio in wt% (content ratio) may be increased.

In an embodiment, the fructooligosaccharide of the present disclosed may be produced by a reaction using a reaction substrate (e.g., sugar (sucrose)) and a nucleic acid molecule encoding the enzyme protein and/or a recombinant microorganism expressing the enzyme protein (specifically, a sugar conversion reaction), and may be included in reaction products and/or conversion products (specifically, a sugar conversion product) resulting from the reaction.

The reaction (specifically, sugar conversion reaction) may be carried out under temperature conditions of 30 to 65 °C, 30 to 60 °C, 30 to 55 °C, 30 to 50 °C, 30 to 45 °C, 35 to 65 °C, 35 to 60 °C, 35 to 55 °C, 35 to 50 °C, or 35 to 45 °C, for example, 40 °C, under conditions of pH of 4.5 to 8.0, pH 4.5 to 7.5, pH 4.5 to 7.0, pH 5 to 8.0, pH 5 to 7.5, pH 5 to 7.0, pH 5.5 to 8.0, pH 5.5 to 7.5, pH 5.5 to 7.0, pH 6 to 8.0, pH 6 to 7.5, pH 6 to 7.0, pH 6.5 to 8.0, pH 6.5 to 7.5, or pH 6.5 to 7.0, for example, pH 7.0, and/or under time conditions of 10 minutes to 3 hours, 10 minutes to 2 hours, 10 minutes to 1 hour, 10 minutes to 40 minutes, 10 minutes to 30 minutes, 20 minutes to 3 hours, 20 minutes to 2 hours, 20 minutes to 1 hour, 20 minutes to 40 minutes, 20 minutes to 30 minutes, for example, 30 minutes. However, the embodiment is not limited thereto.

The content of nystose and/or fructosylnystose (1-F-fructosyl nystose, GF4) included in the reaction product and/or conversion product may be less than 10 wt%, less than 9 wt%, less than 8 wt%, less than 7 wt%, less than 6 wt%, less than 5 wt%, for example, 6.91 wt%, 4.13 wt%, 3.74 wt% or 3.38 wt%, based on 100 wt% of the total solid content of sugars in the reaction product and/or conversion product. The reaction product and/or conversion product may not include nystose and/or fructosylnystose, and for example, may not include both nystose and fructosylnystose.

The content ratio (specifically, weight ratio) of kestose/nystose (e.g., 1-kestose/nystose) included in the reaction product and/or conversion product may be 3 or more, 4 or more, 5 or more, 6 or more, 7 or more, 8 or more, 9 or more, 10 or more, 11 or more, 12 or more, 13 or more, 3 to 60, 3 to 50, 3 to 40, 4 to 60, 4 to 50, 4 to 40, 5 to 60, 5 to 50, 5 to 40, 6 to 60, 6 to 50, 6 to 40, 7 to 60, 7 to 50, 7 to 40, 8 to 60, 8 to 50, 8 to 40, 9 to 60, 9 to 50, 9 to 40, 10 to 60, 10 to 50, 10 to 40, 11 to 60, 11 to 50, 11 to 40, 12 to 60, 12 to 50, 12 to 40, 13 to 60, 13 to 50, 13 to 40, for example, 6.28, 13.01, 13.33, or 17.15. However, the embodiment is not limited thereto. The ratio may be based on the total solid content wt% of sugars included in the conversion product.

The kestose (e.g., 1-kestose) included in the reaction product and/or conversion product may be included in the content of 43 to 70 wt%, 43 to 65 wt% or 43 to 60 wt%, 50 to 70 wt%, for example, 43.37 wt%, 48.67 wt%, 55.06 wt%, or 57.97 wt%, based on 100 wt% of the total solid content of sugars in the reaction product and/or conversion product. However, the embodiment is not limited thereto.

The content of nystose and/or fructosylnystose, the content ratio (specifically, weight ratio) of kestose/nystose (e.g., 1-kestose/nystose), and/or the kestose content may be measured by performing a reaction (specifically, a sugar conversion reaction) using a substrate (e.g., sugar (sucrose)) and a nucleic acid molecule encoding the enzyme protein and/or a recombinant microorganism expressing the enzyme protein, and specifically, may be measured by performing a sugar conversion reaction using an enzyme protein having β-fructofuranosidase activity having 1000 unit activity for sucrose (specifically, a sucrose solution) at a concentration of 250 g/L or 500 g/L. The reaction may be, for example, performed by mixing 150 mM mcilvaine buffer (pH 5.0), 250 g/L sucrose substrate solution, and 1000 units of the enzyme protein in a volume ratio of 2:2:1 or 100 mM phosphate buffer (pH 7.0), 500 g/L sucrose substrate solution, and 1000 units of the enzyme protein in a volume ratio of 4:4:1. The enzyme protein used in the sugar conversion reaction may be in the form of at least one selected from the group consisting of a recombinant microorganism (specifically, a microorganism of the genus *Pichia*) expressing the enzyme protein, a culture of the microorganism, a supernatant obtained from the culture, and an extract thereof.

The reaction may be carried out under temperature conditions of 30 to 65 °C, 30 to 60 °C, 30 to 55 °C, 30 to 50 °C, 30 to 45 °C, 35 to 65 °C, 35 to 60 °C, 35 to 55 °C, 35 to 50 °C, or 35 to 45 °C, for example, 40 °C, and/or under conditions of 10 minutes to 3 hours, 10 minutes to 2 hours, 10 minutes to 1 hour, 10 minutes to 40 minutes, 10 minutes to 30 minutes, 20 minutes to 3 hours, 20 minutes to 2 hours, 20 minutes to 1 hour, 20 minutes to 40 minutes, 20 minutes to 30 minutes, for example, 30 minutes.

According to an embodiment of the present disclosure, a nucleic acid molecule (e.g., DNA) encoding the enzyme protein according to the present disclosure, a recombinant vector including the same, and/or a transformant including the nucleic acid molecule or recombinant vector are provided.

Another embodiment of the present disclosure is interpreted to include a nucleic acid molecule encoding the enzyme protein as well as a sequence that exhibits substantial identity to the nucleotide sequence of the nucleic acid molecule. The substantial identity refers to a nucleotide sequence that exhibits at least 98 % identity or homology when the nucleotide sequence of the present disclosure is aligned with any other sequence as closely as possible and the aligned sequences are analyzed by using an algorithm commonly used in the art.

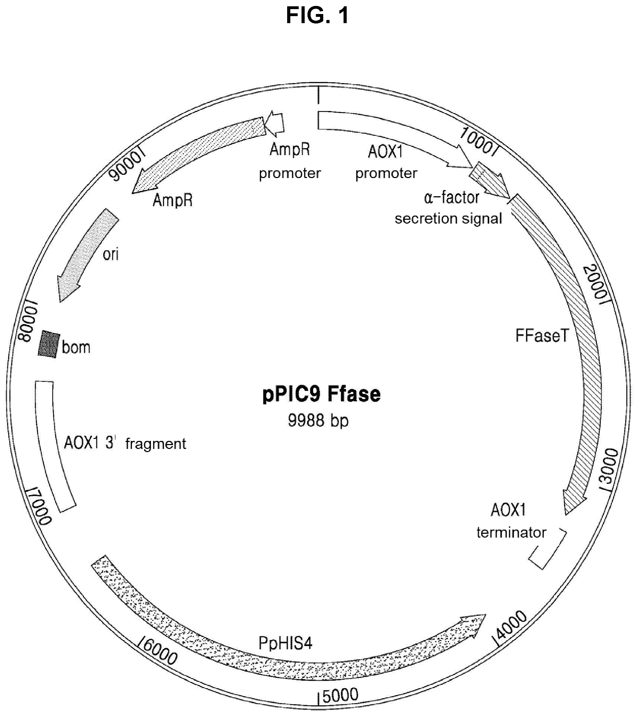

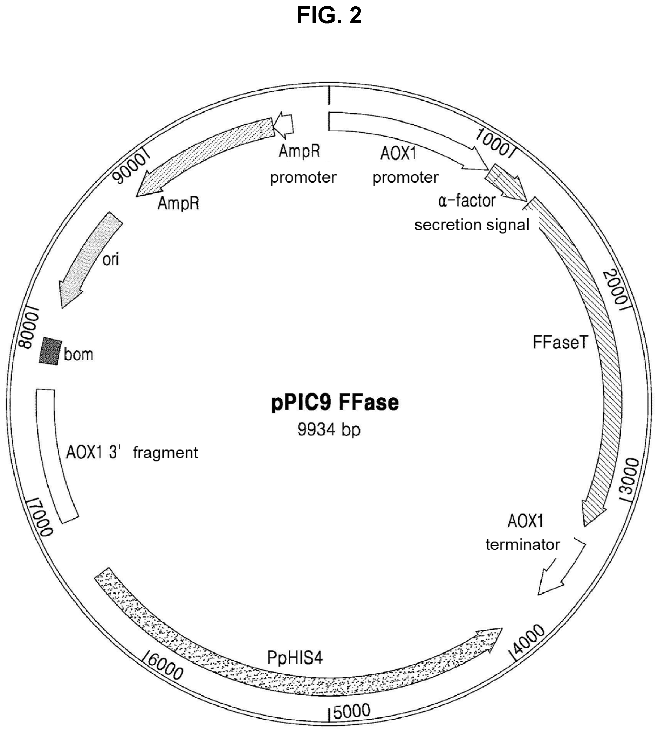

The nucleic acid molecule may be used by itself or in the form of a recombinant vector including the nucleic acid molecule. The above recombinant vector means a recombinant nucleic acid molecule containing a desired gene sequence and an appropriate nucleic acid sequence essential for expressing the gene sequence operably linked in a specific host organism, wherein the appropriate nucleic acid sequence may be a transcription and translation terminator, a transcription and translation initiation sequence, and a promoter useful for regulating expression of a specific target nucleic acid. The vector system may be constructed as a vector for cloning or a vector for expression through various methods widely known in the art. An embodiment of a preferred vector according to the present disclosure may include a nucleic acid molecule encoding the enzyme protein according to the present disclosure and a fungal promoter, for example, a promoter of a strain of the genus *Pichia* or a strain of the genus *Aspergillus*. An example of the recombinant vector according to the present disclosure may be a recombinant vector having the cleavage map of FIG. 1 and/or FIG. 2 and/or a vector in which a mutation has occurred in the recombinant vector.

### Recombinant microorganism including mutant enzyme protein

Another preferred embodiment of the present disclosure provides a recombinant microorganism (e.g., yeast and molds) including a nucleic acid molecule encoding the enzyme protein or a vector including the same, and/or expressing the enzyme protein, and specifically provides a recombinant microorganism (e.g., yeast and mold) expressing the enzyme protein, which is transformed with a recombinant expression vector including a nucleotide sequence encoding the enzyme protein.

The microorganism (specifically, a microorganism to be transformed that is capable of stably and continuously cloning and/or expressing a vector) may be any microorganism without a particular limitation as long as it can overexpress the active form of the enzyme protein. For example, the microorganism may be at least one selected from the group consisting of a strain of the genus *Pichia* (e.g., *P. pastoris*), a strain of the genus *Aspergillus* (e.g., *A. niger* or *A. oryzae*), a strain of the genus *Saccharomyces* (e.g., *S. cerevisiae*), and a strain of the genus *Yarrowia* (e.g., *Y. lipolytica*), and may be preferably the strain of the genus *Pichia*, but is not limited thereto.

Methods for transformation by using the vector, such as fusion of bacterial protoplasts, electroporation, projectile bombardment, and infection using a viral vector, are not particularly limited, and may include those known in the art.

As described above, the enzyme protein has excellent kestose conversion ability, which converts sucrose into kestose, and a low nystose production rate. Therefore, the enzyme protein or the recombinant microorganism expressing the same may produce a high content of kestose.

### Composition for producing sugar conversion product including kestose, including mutant enzyme protein, and/or method of producing sugar conversion product including kestose

Another embodiment of the present disclosure relates to a composition for producing a sugar conversion product including kestose (e.g., 1-kestose) from a substrate including sucrose, and/or a method of producing a sugar conversion product including kestose (e.g., 1-kestose) from a substrate including sucrose, the composition including at least one selected from the group consisting of the enzyme protein, a recombinant microorganism expressing the enzyme protein, a cell of the microorganism, a cell lysate of the microorganism, a culture of the microorganism, a supernatant obtained from the culture, and an extract thereof. The composition for producing a sugar conversion product including kestose may be brought into contact with a substrate or medium including sucrose to produce a high content of kestose. The substrate or medium may include sucrose in any form that sucrose may have. For example, it may include sucrose solids, or may be in the form of a solution containing sucrose. However, an embodiment is not limited thereto.

The culture includes an enzyme protein produced from the recombinant microorganism, and in this regard, may include the recombinant microorganism or be in a cell-free form not including the microorganism. The lysate refers to a lysate obtained by lysing the recombinant microorganism or a supernatant obtained by centrifuging the lysate, and includes an enzyme protein produced from the recombinant microorganism.

In the present specification, unless otherwise stated, the recombinant microorganism used in the production of kestose is used to refer to include at least one selected from the group consisting of a cell of the microorganism, a cell lysate of the microorganism, a culture of the microorganism, a supernatant obtained from the cultures, and an extract thereof. The cells may be obtained by centrifugation, filtration, and the like on a culture of the microorganism. In addition, the resulting cells may be homogenized and centrifuged to obtain a supernatant, and an enzyme protein may be obtained from the supernatant or by fractionation or separation and purification through centrifugation of the supernatant.

Culturing of the recombinant microorganism may be carried out in a medium and under culture conditions easily selected by a person skilled in the art to which the present disclosure pertains, depending on the characteristics of the microorganism used. Culturing methods may include any culture method known in the art, such as batch, continuous, and fed-batch culture methods, but are not limited thereto. The medium used for culturing includes any host cell including *E. coli* and any culture medium, solution, solid, semi-solid, or rigid support that may support or include cell contents.

Regarding the production method, when a recombinant microorganism is used, the cell concentration of a strain used may be 0.1 mg (dry cell weight (dcw))/ml or more, for example, 0.1 to 100 mg (dcw)/ml, or 0.1 to 50 mg (dcw)/ml, based on the total reaction product.

In an embodiment, the method for producing a sugar conversion product including kestose may include reacting a substrate including sucrose with at least one selected from the group consisting of a recombinant microorganism expressing the enzyme protein, a cell of the microorganism, a cell lysate of the microorganism, a culture of the microorganism, a supernatant obtained from the culture, and an extract thereof. The reaction may include, although not limited thereto, contacting a medium including sucrose with at least one selected from the group consisting of a recombinant microorganism expressing the enzyme protein, cells of the microorganism, a cell lysate of the microorganism, a culture of the microorganism, a supernatant obtained from the culture, and an extract thereof.

In another embodiment, the method of producing a sugar conversion product including kestose may include reacting a substrate including sucrose with a recombinant microorganism expressing the enzyme protein or an enzyme protein isolated from the recombinant microorganism. The method of producing a sugar conversion product including kestose may further include, prior to the reacting, culturing a recombinant microorganism expressing the enzyme protein and/or recovering a sugar conversion product.

The reacting using the substrate including sucrose may preferably be performed under optimal activation conditions of the enzyme protein. That is, it may be performed under temperature conditions of 30 to 65 °C, 30 to 60 °C, 30 to 55 °C, 30 to 50 °C, 30 to 45 °C, 35 to 65 °C, 35 to 60 °C, 35 to 55 °C, 35 to 50 °C, or 35 to 45 °C, for example, 40 °C and under conditions of pH 4.5 to 8.0, pH 4.5 to 7.5, pH 4.5 to 7.0, pH 5 to 8.0, pH 5 to 7.5, pH 5 to 7.0, pH 5.5 to 8.0, pH 5.5 to 7.5, pH 5.5 to 7.0, pH 6 to 8.0, pH 6 to 7.5, pH 6 to 7.0, pH 6.5 to 8.0, pH 6.5 to 7.5 or pH 6.5 to 7.0, for example, pH 7.0, and/or may be performed for 10 minutes to 3 hours, 10 minutes to 2 hours, 10 minutes to 1 hour, 10 minutes to 40 minutes, 10 minutes to 30 minutes, 20 minutes to 3 hours, 20 minutes to 2 hours, 20 minutes to 1 hour, 20 minutes to 40 minutes, 20 minutes to 30 minutes, for example, 30 minutes.

In the method of producing a sugar conversion product including kestose, for efficient kestose production, the concentration of sucrose used as a substrate may be, although not limited thereto, 100 to 1,000 g/L, 100 to 900 g/L, 100 to 800 g/L, 100 to 700 g/L, 100 to 600 g/L, 100 to 550 g/L, 200 to 1,000 g/L, 200 to 900 g/L, 200 to 800 g/L, 200 to 700 g/L, 200 to 600 g/L, 200 to 550 g/L, for example, 250 g/L or 500 g/L, based on the total reaction substrate solution. Specifically, the reaction may be, for example, performed by mixing 150 mM mcilvaine buffer (pH 5.0), 250 g/L sucrose substrate solution, and 1000 units of the enzyme protein in a volume ratio of 2:2:1 or 100 mM phosphate buffer (pH 7.0), 500 g/L sucrose substrate solution, and 1000 units of the enzyme protein in a volume ratio of 4:4:1. The enzyme protein may refer to, although not limited thereto, at least one selected from the group consisting of a culture of the recombinant microorganism, i.e., a microorganism in which the reaction is performed, a supernatant obtained from the culture, and an extract thereof.

Another embodiment provides the method of producing a sugar conversion product including kestose, the method including reacting a substrate including sucrose with at least one selected from the group consisting of the enzyme protein, a recombinant microorganism expressing the enzyme protein, a cell of the microorganism, a cell lysate of the microorganism, a culture of the microorganism, a supernatant obtained from the culture, and an extract thereof.

The method of producing a sugar conversion product including kestose may further include isolating, recovering, and/or purifying a sugar conversion product including kestose from at least one selected from the group consisting of the recombinant microorganism reacted with the substrate including sucrose, a cell of the microorganism, a cell lysate of the microorganism, a culture of the microorganism, a supernatant obtained from the culture, and an extract thereof.

An embodiment of the present disclosure provides a sugar conversion composition biologically produced from the substrate including sucrose and including kestose (e.g., 1-kestose), wherein the content of kestose is 40 wt% or more and the content of nystose is less than 10 wt%, based on 100 wt% of the total solid content of sugars included in the sugar conversion composition.

Such a biological method (specifically, an enzymatic method) may involve an enzyme protein, a nucleic acid molecule encoding the enzyme protein, or a vector including the same, and/or may include reacting a substrate with at least one selected from the group consisting of a recombinant microorganism expressing the enzyme protein, a cell of the microorganism, a cell lysate of the microorganism, a culture of the microorganism, a supernatant obtained from the culture, and an extract thereof to prepare kestos, and specifically may include a method of producing a sugar conversion product including kestose, wherein the involvememt of the enzyme protein and the nucleic acid molecule encoding the enzyme protein and/or the recombinant microorganism expressing the enzyme protein, the cell of the microorganism, the cell lysate of the microorganism, the culture of the microorganism, the supernatant obtained from the culture, and the extract thereof, the production method, and the like are as described above.

The content of nystose and/or fructosylnystose (1-F-fructosylnystose, GF4) included in the sugar conversion product may be less than 10 wt%, less than 9 wt%, less than 8 wt%, less than 7 wt%, less than 6 wt%, or less than 5 wt%, for example, 6.91 wt%, 4.13 wt%, 3.74 wt%, or 3.38 wt%, based on 100 wt% of the total solid content of sugars in the sugar conversion product, and the nystose and/or the fructosylnystose may not be included. However, the embodiment is not limited thereto.

The content ratio (specifically, weight ratio) of kestose/nystose (e.g., 1-kestose/nystose) included in the sugar conversion product may be 3 or more, 4 or more, 5 or more, 6 or more, 7 or more, 8 or more, 9 or more, 10 or more, 11 or more, 12 or more, 13 or more, 3 to 60, 3 to 50, 3 to 40, 4 to 60, 4 to 50, 4 to 40, 5 to 60, 5 to 50, 5 to 40, 6 to 60, 6 to 50, 6 to 40, 7 to 60, 7 to 50, 7 to 40, 8 to 60, 8 to 50, 8 to 40, 9 to 60, 9 to 50, 9 to 40, 10 to 60, 10 to 50, 10 to 40, 11 to 60, 11 to 50, 11 to 40, 12 to 60, 12 to 50, 12 to 40, 13 to 60, 13 to 50, 13 to 40, for example, 6.28, 13.01, 13.33, or 17.15. However, the embodiment is not limited thereto. The ratio may be based on the total solid content wt% of sugars included in the sugar conversion product.

The kestose (e.g., 1-kestose) included in the sugar conversion product may be included in the content of 43 to 70 wt%, 43 to 65 wt% or 43 to 60 wt%, 50 to 70 wt%, for example, 43.37 wt%, 48.67 wt%, 55.06 wt%, or 57.97 wt%, based on 100 wt% of the total solid content of sugars in the sugar conversion product. However, the embodiment is not limited thereto.

The content of nystose and/or fructosylnystose, the content ratio (specifically, weight ratio) of kestose/nystose (e.g., 1-kestose/nystose), and/or the content of kestose may be measured after performing the reacting of the substrate including sucrose with at least one selected from the group consisting of the enzyme protein, the recombinant microorganism expressing the enzyme protein, the cell of the microorganism, the cell lysate of the microorganism, the culture of the microorganism, the supernatant obtained from the culture, and the extract thereof, in the method of producing the sugar conversion product. Specifically, the substrate including sucrose may be included at a concentration of 250 g/L or 500 g/L, and the substance reacted with the substrate may have an activity of 1000 units. The reaction may be, for example, performed by mixing 150 mM mcilvaine buffer (pH 5.0), 250 g/L sucrose substrate solution, and 1000 units of at least one selected from the group consisting of the enzyme protein, the recombinant microorganism expressing the enzyme protein, the cell of the microorganism, the lysate of the microorganism, the culture of the microorganism, the supernatant obtained from the culture, and the extract thereof, in a volume ratio of 2:2:1, or by mixing 100 mM phosphate buffer (pH 7.0), 500 g/L sucrose substrate solution, and 1000 units of at least one selected from the group consisting of the enzyme protein, the recombinant microorganism expressing the enzyme protein, the cell of the microorganism, the lysate of the microorganism, the culture of the microorganism, the supernatant obtained from the culture, and the extract thereof, in a volume ratio of 4:4:1.

The reaction using the substrate may be carried out under temperature conditions of 30 to 65 °C, 30 to 60 °C, 30 to 55 °C, 30 to 50 °C, 30 to 45 °C, 35 to 65 °C, 35 to 60 °C, 35 to 55 °C, 35 to 50 °C, or 35 to 45 °C, for example, 40 °C, and/or time conditions of 10 minutes to 3 hours, 10 minutes to 2 hours, 10 minutes to 1 hour, 10 minutes to 40 minutes, 10 minutes to 30 minutes, 20 minutes to 3 hours, 20 minutes to 2 hours, 20 minutes to 1 hour, 20 minutes to 40 minutes, 20 minutes to 30 minutes, for example, 30 minutes.

The sugar conversion composition includes kestose, sucrose and glucose, and may further include at least one selected from the group consisting of fructose, nystose and fructosylnystose.

In a preferred embodiment, the sugar conversion composition may further include 0 to 10 wt% of fructose, 10 to 30 wt% of glucose, and 10 to 30 wt% of sucrose, based on 100 wt% of the composition.

Another embodiment of the present disclosure provides use of the composition for producing a sugar conversion product including kestose from a substrate including sucrose, the composition including at least one selected from the group consisting of the enzyme protein, a recombinant microorganism expressing the enzyme protein, a cell of the microorganism, a cell lysate of the microorganism, a culture of the microorganism, a supernatant obtained from the culture, and an extract thereof.

Another embodiment of the present disclosure provides use of the enzyme protein, a recombinant microorganism expressing the enzyme protein, a cell of the microorganism, a cell lysate of the microorganism, a culture of the microorganism, a supernatant obtained from the culture, and an extract thereof, for preparing a composition for producing a sugar conversion product including kestose from a substrate including sucrose.

The enzyme protein, the recombinant microorganism, the microbial cell, the lysate, the culture, the supernatant, the extract, the production of a sugar conversion product, and the like are as described above.

### Advantageous Effects of Disclosure

The present disclosure relates to: an enzyme for producing kestose, the enzyme having a high kestose conversion rate and a low by-product production rate; a strain expressing the enzyme; and a method of producing kestose by using the enzyme or the strain, whereby the purification cost of kestose can be reduced and high-purity kestose can be produced.

### Brief Description of Drawings

FIG. 1 is a cleavage map of a vector including the β-fructofuranosidase gene, where no mutation has occurred, for the purpose of introducing the *fopA* gene into a *Pichia* strain.
FIG. 2 is a cleavage map of a vector including a mutant enzyme for the β-fructofuranosidase gene, for the purpose of introducing the *fopA* gene into a Pichia strain.
FIG. 3 is a graph showing a ratio of kestose and nystose in a sugar conversion product prepared by a strain into which the FFase-encoding gene is introduced, depending on whether the FFase enzyme gene has been mutated and according to mutation positions.

### Mode for the Invention

The present disclosure will be described in more detail with reference to the following examples, but the scope of the present disclosure is not intended to be limited to the following examples.

### Example 1. Acquisition of fopA gene and development of expression system

### Example 1-1. Acquisition of fopA gene from Aspergillus

It was confirmed in KR 10-2018-0078086 that a microorganism of the genus *Aspergillus* (Accession No. KCTC13139BP) producing fructooligosaccharides had high productivity of kestose as compared to a wild-type microorganism of the genus *Aspergillus*. Accordingly, the genomic DNA of the microorganism of the genus *Aspergillus* with increased productivity was acquired, and the fopA gene sequence (encoding β-fructofuranosidase) was identified.

Specifically, gDNA was acquired by using a genomic DNA prep kit (manufactured by Promega) for the cultured cells of the microorganism of Accession No: KCTC13139BP, and PCR was performed on the gDNA by using the primer pairs of SEQ ID NOs: 8 and 9 shown in Table 1 and by repeating a cycle of 94 °C for 30 seconds, 60 °C for 1 minute, and 72 °C for 4 minutes 30 times to obtain an amplified product of the *fopA* gene (encoding beta-fructofuranosidase (FFase)).

**[Table 1]**

| Primer name | Sequence (5' -> 3') | SEQ ID NO: |
|---|---|---|
| FFst-F | gctacggatccgagctccgtcggcgaaaccc | 8 |
| FFst-R | cgtagcaagcttcccgggccgatgtcttcacag | 9 |
| FFaseEco-F | gccgtagaattcatgaagctcaccactaccacc | 10 |
| FFaseEco-R | gctcgagaattctcaatttctctccggccaggc | 11 |
| mFFaseEco-F | gccgtagaattcatgtcataccacctggacaccac | 12 |

The amplified gene product thus acquired was treated with restriction enzymes, BamHI/XhoI, and then linked to pBluescript SK (+) (Stratagene) treated with the same restriction enzymes to clone and obtain the FFase-encoding gene. As a result of confirming the acquired gene sequence, it was confirmed that the 459th amino acid of the *fopA* gene was mutated from arginine to threonine.

The amino acid sequence (SEQ ID NO: 2) of the β-fructofuranosidase-encoding gene (FFase-encoding gene) of the wild-type microorganism of the genus *Aspergillus* and the amino acid sequence (SEQ ID NO: 4) of the β-fructofuranosidase-encoding gene (FFaseT-encoding gene) of the microorganism of the genus *Aspergillus* (Accession No: KCTC13139BP) with increased productivity are shown in Table 2.

**[Table 2]**

| Type | Amino acid sequence (N -> C) / Nucleic acid sequence (5' -> 3') | SEQ ID NO: |
|---|---|---|
| FFase_amin o acids sequence | SYHLDTTAPPPTNLSTLPNNTLFHVWRPRAHILPAEGQIGDPCA HYTDPSTGLFHVGFLHDGDGIAGATTANLATYTDTSDNGSFLIQ PGGKNDPVAVFDGAVIPVGVNNTPTLLYTSVSFLPIHWSIPYTR GSETQSLAVARDGGRRFDKLDQGPVIADHPFAVDVTAFRDPFV FRSAKLDVLLSLDEEVARNETAVQQAVDGWTEKNAPWYVAVS GGVHGVGPAQFLYRQNGGNASEFQYWEYLGEWWQEATNSS WGDEGTWAGRWGFNFETGNVLFLTEEGHDPQTGEVFVTLGT EGSGLPIVPQVSSIHDMLWAAGEVGVGSEQEGAKVEFSPSMA GFLDWGFSAYAAAGKVLPASSAVSKTSGVEVDRYVSFVWLTG DQYEQADGFPTAQQGWTGSLLLPRELKVQTVENVVDNELVRE EGVSWVVGESDNQTARLRTLGITIARETKAALLANGSVTAEED RTLQTAAVVPFAQSPSSKFFVLTAQLEFPASARSSPLQSGFEIL ASELERTAIYYQFSNESLVVDRSQTSAAAPTNPGLDSFTESGKL RLFDVIENGQEQVETLDLTVVVDNAVVEVYANGRFALSTWARS WYDNSTQIRFFHNGEGEVQFRNVSVSEGLYNAWPERN | 1 |
| FFase_full_a mino acids sequence | MKLTTTTLALATGAAAAEASYHLDTTAPPPTNLSTLPNNTLFHV WRPRAHILPAEGQIGDPCAHYTDPSTGLFHVGFLHDGDGIAGA TTANLATYTDTSDNGSFLIQPGGKNDPVAVFDGAVIPVGVNNT PTLLYTSVSFLPIHWSIPYTRGSETQSLAVARDGGRRFDKLDQ GPVIADHPFAVDVTAFRDPFVFRSAKLDVLLSLDEEVARNETAV QQAVDGWTEKNAPWYVAVSGGVHGVGPAQFLYRQNGGNAS EFQYWEYLGEWWQEATNSSWGDEGTWAGRWGFNFETGNVL | 2 |
| | FLTEEGHDPQTGEVFVTLGTEGSGLPIVPQVSSIHDMLWAAGE VGVGSEQEGAKVEFSPSMAGFLDWGFSAYAAAGKVLPASSAV SKTSGVEVDRYVSFVWLTGDQYEQADGFPTAQQGWTGSLLLP RELKVQTVENVVDNELVREEGVSWVVGESDNQTARLRTLGITI ARETKAALLANGSVTAEEDRTLQTAAVVPFAQSPSSKFFVLTA QLEFPASARSSPLQSGFEILASELERTAIYYQFSNESLVVDRSQ TSAAAPTNPGLDSFTESGKLRLFDVIENGQEQVETLDLTVVVDN AVVEVYANGRFALSTWARSWYDNSTQIRFFHNGEGEVQFRNV SVSEGLYNAWPERN | |
| FFaseT_ami no acids sequence | SYHLDTTAPPPTNLSTLPNNTLFHVWRPRAHILPAEGQIGDPCA HYTDPSTGLFHVGFLHDGDGIAGATTANLATYTDTSDNGSFLIQ PGGKNDPVAVFDGAVIPVGVNNTPTLLYTSVSFLPIHWSIPYTR GSETQSLAVARDGGRRFDKLDQGPVIADHPFAVDVTAFRDPFV FRSAKLDVLLSLDEEVARNETAVQQAVDGWTEKNAPWYVAVS GGVHGVGPAQFLYRQNGGNASEFQYWEYLGEWWQEATNSS WGDEGTWAGRWGFNFETGNVLFLTEEGHDPQTGEVFVTLGT EGSGLPIVPQVSSIHDMLWAAGEVGVGSEQEGAKVEFSPSMA GFLDWGFSAYAAAGKVLPASSAVSKTSGVEVDRYVSFVWLTG DQYEQADGFPTAQQGWTGSLLLPRELKVQTVENVVDNELVRE EGVSWVVGESDNQTATLRTLGITIARETKAALLANGSVTAEEDR TLQTAAVVPFAQSPSSKFFVLTAQLEFPASARSSPLQSGFEILA SELERTAIYYQFSNESLVVDRSQTSAAAPTNPGLDSFTESGKLR LFDVIENGQEQVETLDLTVVVDNAVVEVYANGRFALSTWARSW YDNSTQIRFFHNGEGEVQFRNVSVSEGLYNAWPERN | 3 |
| FFaseT_full_ amino acids sequence | MKLTTTTLALATGAAAAEASYHLDTTAPPPTNLSTLPNNTLFHV WRPRAHILPAEGQIGDPCAHYTDPSTGLFHVGFLHDGDGIAGA TTANLATYTDTSDNGSFLIQPGGKNDPVAVFDGAVIPVGVNNT PTLLYTSVSFLPIHWSIPYTRGSETQSLAVARDGGRRFDKLDQ GPVIADHPFAVDVTAFRDPFVFRSAKLDVLLSLDEEVARNETAV QQAVDGWTEKNAPWYVAVSGGVHGVGPAQFLYRQNGGNAS EFQYWEYLGEWWQEATNSSWGDEGTWAGRWGFNFETGNVL FLTEEGHDPQTGEVFVTLGTEGSGLPIVPQVSSIHDMLWAAGE VGVGSEQEGAKVEFSPSMAGFLDWGFSAYAAAGKVLPASSAV | 4 |
| | SKTSGVEVDRYVSFVWLTGDQYEQADGFPTAQQGWTGSLLLP RELKVQTVENVVDNELVREEGVSWVVGESDNQTATLRTLGITI ARETKAALLANGSVTAEEDRTLQTAAVVPFAQSPSSKFFVLTA QLEFPASARSSPLQSGFEILASELERTAIYYQFSNESLVVDRSQ TSAAAPTNPGLDSFTESGKLRLFDVIENGQEQVETLDLTVVVDN AVVEVYANGRFALSTWARSWYDNSTQIRFFHNGEGEVQFRNV SVSEGLYNAWPERN | |
| FFaseT_pichi a DNA sequence | ccgggtttctggactgggggttcagcgcctacgctgcggcgggcaaggtgctgccggcc agctcggcggtgtcgaagaccagcggcgtggaggtggatcggtatgtctcgttcgtctggt tgacgggcgaccagtacgagcaggcggacgggttccccacggcccagcaggggtgg acggggtcgctgctgctgccgcgcgagctgaaggtgcagacggtggagaacgtcgtcg acaacgagctggtgcgcgaggagggcgtgtcgtgggtggtgggggagtcggacaacc agacggccacgctgcgcacgctggggatcacgatcgcccgggagaccaaggcggcc ctgctggccaacggctcggtgaccgcggaggaggaccgcacgctgcagacggcggc cgtcgtgccgttcgcgcaatcgccgagctccaagttcttcgtgctgacggcccagctgga gttccccgcgagcgcgcgctcgtccccgctccagtccgggttcgaaatcctggcgtcgga gctggagcgcacggccatctactaccagttcagcaacgagtcgctggtcgtcgaccgca gccagactagtgcggcggcgcccacgaaccccgggctggatagctttactgagtccgg caagttgcggttgttcgacgtgatcgagaacggccaggagcaggtcgagacgttggatc tcactgtcgtcgtggataacgcggttgtcgaggtgtatgccaacgggcgctttgcgttgagc acctgggcgagatcgtggtacgacaactccacccagatccgcttcttccacaacggcga gggcgaggtgcagttcaggaatgtctccgtgtcggaggggctctataacgcctggccgg agagaaattga | 5 |

(wherein, in Table 2, the sequences including mutations are indicated in both bold and underlined.)

### Example 1-2. Manufacture of expression system for fopA gene expression in Pichia pastoris

When the *Aspergillus*-derived FFase is expressed in a eukaryotic expression system, its enzymatic activity may be confirmed to be similar to that of existing cells, and thus, for the purpose of mutation, the FFase-encoding gene and the FFaseT-encoding gene of Example 1-1 were each commissioned to Bioneer to be engineered into a form suitable for expression in *Pichia* (*Pichia* codon- optimized). The nucleic acid sequence information of the FFaseT-encoding gene which underwent codon optimization is shown in Table 2 (SEQ ID NO: 5).

To develop an enzyme for high-yield production of kestose, a vector including the aforementioned codon-optimized gene was constructed and inserted into the eukaryotic strain of *P. pastoris* to verify its expression.

Specifically, each of the previously prepared genes was amplified by using the primer pairs of SEQ ID NOs: 10 and 11 shown in Table 1. Additionally, to prepare a mature form of the enzyme expressed by the genes in which the N-terminal 19 amino acids have been removed, the genes were amplified by using the primer pairs of SEQ ID NOs: 11 and 12.

The amplified mature form of the genes were purified and treated with a restriction enzyme, EcoRI. In addition, the pPIC9 vector (SnapGene) was also treated with EcoRI, sequentially treated with alkaline phosphatase, and then subcloned by ligation with the amplified genes in their mature forms.

It was confirmed that the subcloned plasmids had been correctly inserted through sequence analysis, and the pPIC9-FFase vector with the FFase-encoding gene introduced and the pPIC9-FFaseT vector with the FFaseT-encoding gene introduced were prepared. The vector maps of the pPIC9-FFase vector and pPIC9-FFaseT vector prepared by the process above are shown in FIGS. 1 and 2, respectively, and the amino acid sequences of the FFase enzyme (SEQ ID NO: 1) and FFaseT enzyme (SEQ ID NO: 3) manufactured in their mature forms are shown in Table 2.

### Example 2. Preparation of P. pastoris strain expressing mutant enzyme

### Example 2-1. Preparation of mutant gene

To engineer the enzyme for increased kestos production by FFaseT, error-prone PCR was performed on the *fopA* gene within the pPIC9-FFaseT vector of Example 1-2 to induce mutations.

Specifically, the pPIC9-FFaseT vector was used as a PCR template, and 5 ng of the template was prepared. PCR was performed by using 1 to 2.5 mM of dCTP and dTTP, 0.1 to 2.5 mM of dATP and dGTP, 1 to 5 mM of MgCl₂, and 0.5 to 1 mM of MnCl₂. By repeating a cycle of 94 °C for 30 seconds, 59 °C for 45 seconds, and 72 °C for 3 minutes and 50 seconds 30 times, the additionally mutated FFaseT-encoding gene (FFaseT mutant gene) was obtained.

The additionally mutated FFaseT mutant gene was treated with EcoRI and subcloned into the pPIC9 vector in substantially the same manner as in Example 1-2 to prepare the pPIC9_FFase mutants vector including the mutant gene.

### Example 2-2. Transformation of FFaseT-encoding gene into P. pastoris

To integrate the β-fructofuranosidase gene into the chromosome of *P. pastoris* GS115 (Invitrogen) and enable protein expression, the purified DNA was transformed into the *Pichia* after cleaving the pPIC9-FFase vector and the pPIC9-FFaseT vector of Example 1-2 and the pPIC9_FFase mutants vector of Example 2-1 with BglII (New England Biolab).

Specifically, to prepare *P. pastoris* competent cells for electroporation, 400 µl of cultured cells of G115 (Invitrogen) were inoculated into 400 mL of yeast extract peptone dextrose (YPD, 10 g/L of yeast extract, 20 g/L of bacto_peptone, and 20 g/L of dextrose), cultured overnight, and collected when the OD600 value was 1 to 1.5.

After collecting the cells, the cells were added to a medium containing 50 mL of YPD and 10 mL of 1 M HEPES, and 1 mL of 1 M DTT was added to the medium. Then, a reaction was allowed at 30 °C for 30 minutes. Following the reaction, the cells were resuspended in an equal volume of ice-cold 1 M sorbitol solution as the culture solution and centrifuged to remove the supernatant. The cells were washed twice by using the same sorbitol solution in a volume equal to 1/2 and 1/10 of the culture solution, and resuspended in 1 mL of 1 M sorbitol solution and divided into 100 µL.

1 to 2 µg of the DNA purified after cleaving the pPIC9-FFase vector and the pPIC9-FFaseT vector of Example 1-2 and the pPIC9_FFase mutants vector of Example 2-1 with BglII (New England Biolab) at 37 °C for 2 to 3 hours was added to 100 µL of the washed *P. pastoris* competent cells, and electroporation was performed thereon to transform the cells by using an electroporator (Bio-Rad) set to the *P. pastoris* (1.5 kv, 25 µF) and supplying a pulse thereto.

### Example 2-3. Selection of strains expressing FFase enzyme

For the transformed cells (strains) in Example 2-2, top agar made with 0.8 % agar (Difco) was spread on an RD-His plate, and the cells cultured thereon at 30 °C. The composition information of the RD-His plate used is shown in Table 3.

Colonies were acquired by culturing the cells for 3 to 5 days on the RD-His plate. The colonies were then equally plated on an MD plate, an MS plate, and an MM plate, respectively, and cultured for 1 to 2 days to identify the growth patterns. The composition information of the plates used is shown in Table 4.

**[Table 3]**

| RD-His plate | |
|---|---|
| Component | g/L |
| Sorbitol | 182 |
| YNB | 1.7 |
| Ammonium sulfate | 5 |
| Glucose | 20 |
| Biotin | 0.04 |
| Amino acids mixture (Histidine drop out) | 0.5 |
| Agar | 20 |

**[Table 4]**

| MD plate | | MS plate | | MM plate | |
|---|---|---|---|---|---|
| Component | g/L | Component | g/L | Component | g/L |
| Yeast nitrogen base (YNB) | 1.7 | Yeast nitrogen base (YNB) | 1.7 | Yeast nitrogen base (YNB) | 1.7 |
| Ammonium sulfate | 5 | Ammonium sulfate | 5 | Ammonium sulfate | 5 |
| Glucose | 20 | Sucrose | 20 | Methanol | 5 |
| Agar | 20 | Agar | 20 | Agar | 20 |

Based on the characteristics that the *P. pastoris* does not grow well in a medium containing sucrose, strains having the FFase enzyme activity may be selected through a process of selecting strains that grew simultaneously in the MS medium containing sucrose and the MM medium which is confirmed to be methylotrophic. Finally, colonies that grew on all plates were selected.

To confirm whether the enzyme has been introduced, integration of the gene into gDNA was confirmed through colony PCR (using Bioneer premix) on the selected colonies.

### Example 3. Confirmation of expression and activity of FFase enzyme

### Example 3-1. Cultivation of strains transformed with FFase enzyme

To confirm the enzyme activity through cultivation of the selected strains among those transformed with the pPIC9-FFase mutants vector in Example 2-3, the selected colonies were inoculated into 50 mL of BMGY medium (Table 5) and cultured at 28 °C and 200 rpm for 24 to 48 hours until the OD600 reached 15 to 20.

The cultured cells were centrifuged at 4000 rpm for 5 minutes to remove the supernatant, and then resuspended in 10 mL of BMMY medium (Table 5) and cultured under the same culture conditions. Additionally, methanol was fed every 24 hours at a concentration of 0.5 (w/v)% to induce expression of the enzyme in the strain. The composition information of the BMGY medium and the BMMY medium used is shown in Table 5.

**[Table 5]**

| BMGY | | BMMY | |
|---|---|---|---|
| Component | - | Component | - |
| Yeast extract | 5 g/L | Yeast extract | 5 g/L |
| Peptone | 10 g/L | Peptone | 10 g/L |
| Phosphate buffer pH 6.0 | 100 mM | Phosphate buffer pH 6.0 | 100 mM |
| Yeast nitrogen base without amino acid (YNB) | 3.4 g/L | Yeast nitrogen base without amino acid (YNB) | 3.4 g/L |
| Ammonium sulfate | 10 g/L | Ammonium sulfate | 10 g/L |
| Biotin | 0.002 g/L | Biotin | 0.002 g/L |
| Glycerol | 10 g/L | Methanol (99.99 % or more) | 5 g/L |

### Example 3-2. Confirmation of FFase enzyme activity

The FFase enzyme activity was confirmed 4 days after the cultivation described in Example 3-1. In the case of the cultured cells, the enzyme is expressed in a form fused to an alpha-mating factor and secreted outside the cells, and thus the supernatant of the cell medium was analyzed to confirm the enzyme activity.

Specifically, for the enzyme activity analysis by using the supernatant, 150 mM of mcilvaine buffer( pH 5.0), 250 g/L of sucrose substrate solution, and the supernatant (1000 units) of the culture solution cultured in the BMMY medium described in Example 3-1 were added in a volume ratio of 2:2:1 and reacted at 40 °C for 30 minutes, 1 hour, or 2 hours, and the reaction solution was quantitatively analyzed through HPLC. The analysis was performed by using a 70 % acetonitrile solution as a mobile phase at a flow rate of 1 mL/min, with an NH2P-50 column (Shodex), and the results are confirmed by analysis using a refractive index detector (RID) at 30 °C.

The ratios of different types of sugars in the conversion product over time were confirmed over the reaction time, specifically the ratio of GF2 (kestose) and GF3 (nystose) converted at 10 to 15% of the reaction substrate, sugar, and the results are shown in Table 6. The numerical unit in Table 6 is the content of each constituent sugar expressed as wt% based on 100 wt% of the total sugar solid content included in the conversion product.

**[Table 6]**

| Reaction time | Fructose | Glucose | Sucrose | GF2 | GF3 | GF4 |
|---|---|---|---|---|---|---|
| 30 min | 4.22 | 25.63 | 12.84 | 39.54 | 16.95 | 0.37 |
| 1 hr | 4.67 | 28.17 | 10.71 | 29.94 | 24.38 | 0.78 |
| 2 hr | 6.09 | 32.82 | 7.72 | 16.14 | 28.78 | 1.94 |

As a result of confirming the ratios of sugars in the conversion product, it was confirmed that, due to the high enzyme activity of the strain, the substrate ratio dropped to 15 % or less within 30 minutes. However, it was confirmed that extending the reaction time led to excessive transfer of fructose, resulting in the increased ratio of GF3 and GF4. These results showed that the ratio of GF2 (kestose) decreased as the enzyme reaction time passed.

### Example 4. Analysis of mutant enzyme expression and activity

### Example 4-1. Selection of mutant enzymes by using TLC

To analyze the enzyme activity of the enzyme in the pPIC9_FFase mutants vector, the strain selected among the *Pichia* strains transformed with the pPIC9_FFase mutants vector of Example 2-3 was cultured in substantially the same manner as in Example 3-1, and reacted in the same manner as in Example 3-2. The reaction was performed at 40 °C for 30 minutes.

Following the reaction, the reaction solution was placed on a thin layer chromatography (TLC) plate (Merck) and developed to confirm the conversion of sucrose according to the enzyme activity. The developing solvent used for TLC confirmation was a solution of acetonitrile/water mixed at a ratio of 85/15, and after the development, the samples were identified by using a solution of N-naphthyldiethyllaurine dihydrochloride. In addition, the *Pichia* strain having the mutant enzyme that was confirmed to use and convert sugar to kestose in the reaction through the development was acquired.

### Example 4-2. Confirmation and selection of mutant enzyme activity

The strain selected among the strains transformed with the FFase or the FFaseT in Example 2-3 and the strain acquired in Example 4-1 were cultured and reacted in substantially the same manner as in Example 4-1.

Following the reaction, the reaction solution was quantitatively analyzed by HPLC analysis in substantially the same manner as in Example 3-2 to confirm the primary activity, and the results are shown in Table 7.

**[Table 7]**

| Enzyme name | Fructose | Glucose | Sucrose | GF2 | GF3 | GF4 |
|---|---|---|---|---|---|---|
| FFase | 4.22 | 25.63 | 12.84 | 39.54 | 16.95 | 0.37 |
| FFaseT | 2.51 | 23.62 | 11.42 | 41.46 | 20.45 | 0.43 |
| FFa mM8 | 1.62 | 17.86 | 29.81 | 43.37 | 6.91 | - |
| FFa m62W | 9.08 | 26.27 | 11.2 | 48.67 | 3.74 | - |

As a result of confirming enzyme activity, the fructose transfer activity of FFaseT with amino acid mutation increased compared to FFase without mutation, and the production of GF2 and GF3 increased. In addition, it showed the characteristic of maintaining a high ratio of GF2 as compared to the wild-type FFase.

In addition, it was difficult to confirm the activity of strains with low expression levels among those obtained in Example 4-1, and the activity of the strains in which the enzyme was expressed was confirmed. As a result of confirming the primary activity, mutant enzymes with high GF2 production activity and low GF3 conversion compared to the FFase and the FFaseT, specifically strains with low GF3 production, were selected. The results of confirming the activity of the selected strains are shown in Table 7.

Specifically, strains expressing mutant enzymes with confirmed mutations of V220A (V239A), H313K (H332K), E329K (E348K), and R440T (R459T) and strains expressing mutant enzymes with confirmed mutations of G62W (G81W), H313K (H332K), and R440T (R459T) were selected. Such mutations refer to mutation information based on the mature form of the enzyme, and mutations in parentheses refer to mutation information based on the non-mature form of the enzyme.

Among the selected enzymes, the enzyme with four mutations was named FFa mM8, and the enzyme with three mutations was named FFa m62W. The amino acid sequence (SEQ ID NO: 6) of FFa mM8 and the amino acid sequence (SEQ ID NO: 7) of FFa m62W are shown in Table 8.

**[Table 8]**

| Amino acid type | Sequence (N → C) | SEQ ID NO. |
|---|---|---|
| FFa_mM8_PR T | SYHLDTTAPPPTNLSTLPNNTLFHVWRPRAHILPAEGQIGDPC AHYTDPSTGLFHVGFLHDGDGIAGATTANLATYTDTSDNGSF LIQPGGKNDPVAVFDGAVIPVGVNNTPTLLYTSVSFLPIHWSIP YTRGSETQSLAVARDGGRRFDKLDQGPVIADHPFAVDVTAFR DPFVFRSAKLDVLLSLDEEVARNETAVQQAVDGWTEKNAPW YVAVSGGAHGVGPAQFLYRQNGGNASEFQYWEYLGEWWQ EATNSSWGDEGTWAGRWGFNFETGNVLFLTEEGHDPQTGE VFVTLGTEGSGLPIVPQVSSIKDMLWAAGEVGVGSEQKGAKV EFSPSMAGFLDWGFSAYAAAGKVLPASSAVSKTSGVEVDRY VSFVWLTGDQYEQADGFPTAQQGWTGSLLLPRELKVQTVEN VVDNELVREEGVSWVVGESDNQTATLRTLGITIARETKAALLA NGSVTAEEDRTLQTAAVVPFAQSPSSKFFVLTAQLEFPASAR SSPLQSGFEILASELERTAIYYQFSNESLVVDRSQTSAAAPTN PGLDSFTESGKLRLFDVIENGQEQVETLDLTVVVDNAVVEVYA NGRFALSTWARSWYDNSTQIRFFHNGEGEVQFRNVSVSEGL YNAWPERN | 6 |
| FFa_m62W_P RT | SYHLDTTAPPPTNLSTLPNNTLFHVWRPRAHILPAEGQIGDPC AHYTDPSTGLFHVGFLHDWDGIAGATTANLATYTDTSDNGSF LIQPGGKNDPVAVFDGAVIPVGVNNTPTLLYTSVSFLPIHWSIP YTRGSETQSLAVARDGGRRFDKLDQGPVIADHPFAVDVTAFR DPFVFRSAKLDVLLSLDEEVARNETAVQQAVDGWTEKNAPW YVAVSGGVHGVGPAQFLYRQNGGNASEFQYWEYLGEWWQ EATNSSWGDEGTWAGRWGFNFETGNVLFLTEEGHDPQTGE | 7 |
| | VFVTLGTEGSGLPIVPQVSSIKDMLWAAGEVGVGSEQEGAKV EFSPSMAGFLDWGFSAYAAAGKVLPASSAVSKTSGVEVDRY VSFVWLTGDQYEQADGFPTAQQGWTGSLLLPRELKVQTVEN VVDNELVREEGVSWVVGESDNQTATLRTLGITIARETKAALLA NGSVTAEEDRTLQTAAVVPFAQSPSSKFFVLTAQLEFPASAR SSPLQSGFEILASELERTAIYYQFSNESLVVDRSQTSAAAPTN PGLDSFTESGKLRLFDVIENGQEQVETLDLTVVVDNAVVEVYA NGRFALSTWARSWYDNSTQIRFFHNGEGEVQFRNVSVSEGL YNAWPERN | |

### Example 4-3. Confirmation of mutant enzyme activity on high-concentration substrate

After confirming the primary activity, the enzymes of the selected strains, FFa mM8 and FFa m62W, were tested for activity on a high-concentration substrate.

Specifically, 100 mM phosphate buffer (pH 7.0), 500 g/L of sucrose solution, and the supernatant (1,000 units) of the culture solution obtained by culturing each of the two strains selected in Example 4-2 in substantially the same manner as in Example 3-1 were added in a volume ratio of 4:4:1 and reacted at 40 °C for 1 hour.

After the reaction, the ratio of the products was confirmed through HPLC analysis in substantially the same manner as in Example 3-2, and the HPLC analysis results of the selected strains are shown in Table 9. The numerical unit in Table 9 is the content of each constituent sugar expressed as wt% based on 100 wt% of the sugar solid content included in the conversion product.

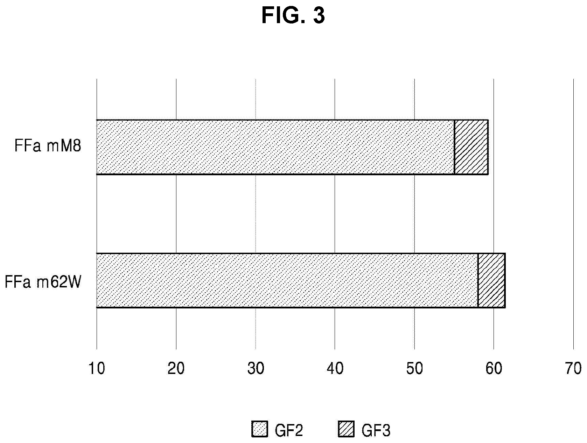

In addition, the results obtained by combining the ratio of GF2 and GF3 of each enzyme with the results of Example 3-2 are shown in FIG. 3.

**[Table 9]**

| Enzyme name | Fructose | Glucose | Sucrose | GF2 | GF3 |
|---|---|---|---|---|---|
| FFa mM8 | 1.78 | 19.31 | 19.71 | 55.06 | 4.13 |
| FFa m62W | 3.05 | 21.63 | 13.71 | 57.97 | 3.38 |

As a result of confirming the enzyme activity, it was confirmed that the mutant enzymes, FFa mM8 and FFa m62W, produced kestose in the solid content of greater 50 wt% and nystose in the content of less than 5 wt%, based on the sugar solid content included in the sugar conversion production, confirming high kestose production activity compared to the FFase enzyme where no highly active mutation converting kestose to nystose has occurred.

In addition, in the case of FFa m62W, it was confirmed that the mature form of FFase (a form from which the 19-amino acid-signal sequence has been removed) was produced, and using this enzyme, a high content of kestose was produced.

In combination with the results of Example 3-2, the GF2/GF3 ratio, which is a measure of the reaction converting to the oligosaccharide kestose, was confirmed to be 2.3 for the wild-type, 13.33 for mM8, and 17.15 for m62W.

Based on the results above, it was confirmed that the enzyme exhibits the characteristic of specifically converting to kestose due to the residue mutations such as G62W (G81W), V220A (V239A), H313K (H332K), E329K (E348K), and R440T (R459T).

## Claims

1. An enzyme protein having β-fructofuranosidase activity, the enzyme protein comprising an amino acid sequence having at least 90 % sequence identity with the amino acid sequence of SEQ ID NO: 1, wherein an amino acid corresponding to at least one amino acid selected from the group consisting of the 62nd, 220th, 313th, 329th, and 440th amino acids from the N-terminus of the amino acid sequence of SEQ ID NO: 1 is mutated.

2. The enzyme protein having β-fructofuranosidase activity of claim 1, wherein the amino acid corresponding to at least one amino acid selected from the group consisting of the 62nd, 220th, 313th, 329th and 440th amino acids from the N-terminus of the amino acid sequence of SEQ ID NO: 1 is substituted with at least one amino acid selected from the group consisting of tryptophan (Trp; W), alanine (Ala; A), lysine (Lys; K), and threonine (Thr; T).

3. The enzyme protein having β-fructofuranosidase activity of claim 2, wherein the substitution comprises at least one selected from the group consisting of a substitution of an amino acid corresponding to glycine (Gly; G) at position 62 with Trp, a substitution of an amino acid corresponding to valine (Val; V) at position 220 with Ala, a substitution of an amino acid corresponding to histidine (His; H) at position 313 with Lys, a substitution of an amino acid corresponding to glutamic acid (Glu; E) at position 329 with Lys, and a substitution of an amino acid corresponding to arginine (Arg; R) at position 440 with Thr, wherein the amino acids are from the N-terminus of the amino acid sequence of SEQ ID NO: 1.

4. The enzyme protein having β-fructofuranosidase activity of claim 1, wherein a content of nystose included in a sugar conversion product prepared by a reaction using the enzyme protein and a substrate including sucrose is less than 10 wt% based on 100 wt% of a total solid content of sugars included in the sugar conversion product.

5. The enzyme protein having β-fructofuranosidase activity of claim 1, wherein a weight ratio of kestose/nystose included in a sugar conversion product prepared by a reaction using the enzyme protein and a substrate including sucrose is 3 to 60.

6. The enzyme protein having β-fructofuranosidase activity of claim 5, wherein the weight ratio is measured by performing a sugar conversion reaction using the enzyme protein having β-fructofuranosidase activity and exhibiting 1000 unit of activity with respect to a sucrose solution at a concentration of 250 g/L or 500 g/L.

7. The enzyme protein having β-fructofuranosidase activity of claim 6, wherein the sugar conversion reaction is performed under:
(1) a time condition of 10 minutes to 3 hours;
(2) a temperature condition of 30 to 65 °C; or
(3) a condition of pH 4.5 to 8.0.

8. The enzyme protein having β-fructofuranosidase activity of claim 6, wherein the enzyme protein used in the sugar conversion reaction is in a form of at least one selected from the group consisting of a microorganism of the genus *Pichia* expressing the enzyme protein, a culture of the microorganism, a supernatant obtained from the culture, and an extract thereof.

9. A nucleic acid molecule encoding the enzyme protein according to any one of claims 1 to 8.

10. A recombinant vector comprising the nucleic acid molecule according to claim 9.

11. A recombinant microorganism comprising the nucleic acid molecule according to claim 9 or a recombinant vector comprising the same.

12. The recombinant microorganism of claim 11, wherein the recombinant microorganism is a strain of the genus *Pichia*, a strain of the genus *Aspergillus*, a strain of the genus *Saccharomyces*, or a strain of the genus *Yarrowia*.

13. A composition for producing a sugar conversion product including kestose from a substrate including sucrose, the composition comprising at least one selected from the group consisting of the enzyme protein according to any one of claims 1 to 8, a recombinant microorganism expressing the enzyme protein, a cell of the microorganism, a cell lysate of the microorganism, a culture of the microorganism, a supernatant obtained from the culture, and an extract thereof.

14. The composition of claim 13, wherein a content of nystose included in the sugar conversion product is less than 10 wt% based on 100 wt% of a total solid content of sugars included in the sugar conversion product.

15. The composition of claim 13, wherein a weight ratio of kestose/nystose included in the sugar conversion product is 3 to 60.

16. The composition of claim 13, wherein a concentration of the substrate including sucrose is 100 to 1,000 g/L.

17. A method of producing a sugar conversion product including kestose, the method comprising reacting a substrate including sucrose with at least one selected from the group consisting of the enzyme protein according to any one of claims 1 to 8, a recombinant microorganism expressing the enzyme protein, a cell of the microorganism, a cell lysate of the microorganism, a culture of the microorganism, a supernatant obtained from the culture, and an extract thereof.

18. A sugar conversion composition, produced biologically from the substrate including sucrose and comprising kestose,
wherein a content of kestose is 40 wt% or more and a content of nystose is less than 10 wt%, based on 100 wt% of a total solid content of sugars included in the sugar conversion composition.

19. The sugar conversion composition of claim 18, further comprising 10 to 30 wt% of glucose and 10 to 30 wt% of sucrose.
